# EUROPEAN PATENT APPLICATION

(11) **EP 2 995 676 A1**
(43) Date of publication of application: **16.03.2016**
(21) Application number: 13884003.8
(22) Date of filing: 06.05.2013
(51) Int. Cl.: C12M 1/34, C12M 1/36, C12M 3/00, C12Q 1/02

(54) **DEVICE FOR ANALYZING CELLS AND MONITORING CELL CULTURING AND METHOD FOR ANALYZING CELLS AND MONITORING CELL CULTURING USING SAME**

(71) Applicant: Optolane Technologies Inc., Bundang-gu Seongnam-si, Gyeonggi-do 463-400 (KR)
(72) Inventor: KIM, Songyi, Seoul 152-700 (KR)
(74) Representative: Samson & Partner Patentanwälte mbB
(86) International application number: PCT/KR2013/003922
(87) International publication number: WO 2014/181897

(57) **Abstract**

The present invention relates to a system for analyzing cells and monitoring cell culture and a method for analyzing cells and monitoring cell culture using the same. The system comprises M x N matrix camera modules disposed below the cell culture unit in which a cell culture vessel is received, and thus can simultaneously analyze changes in the cells being cultured in a multi-well cell culture vessel such as a 96-well plate. Particularly, the system enables a desired portion to be observed by selectively switching on camera modules corresponding to the desired portion. In addition, the system can microscopically observe entire wells or can microscopically observe the region of interest of a single cell at high magnifications, can display dynamic cell images in real time, can overcome the problem of contamination during cell analysis and culture, and can remotely check the state of culture in real time.

## Description

### Technical Field

The present invention relates, in general, to a system for analyzing cells or monitoring cell culture and a method for analyzing cells or monitoring cell culture using the same, and more particularly, to a system for analyzing cells or monitoring cell culture, which comprises M x N matrix camera modules disposed below the cell culture unit in which a cell culture vessel is received, and thus can simultaneously analyze changes (caused by various changes in culture conditions or external environments) in the cells being cultured in a multi-well cell culture vessel such as a 96-well plate, and to a method for analyzing cells and monitoring cell culture using the system for analyzing cells and monitoring cell culture. Particularly, the system according to the present invention enables a desired portion to be observed by selectively switching on only camera modules corresponding to the desired portion, and can microscopically observe entire wells or can microscopically observe the region of interest of a single cell at high magnifications.

### Background Art

Industrial fields and recognized fields to which cultured cells are applied have been extensively expanded. For example, cells for production of recombinant proteins, cells for screening of pharmacological molecules, cells for diagnostic examination or the like, cells for use in toxicity evaluation techniques, cells for restoration therapy and regenerative cell therapy, and the like, have been studied and applied. In particular, stem cells, which have been most actively studied in the bioscience field, differentiate into cells having a certain function depending on environmental and stimulating conditions, and thus it is necessarily required to analyze and track a cell differentiation process during cell culture in order to find the mechanism of action of stem cells or a method for deriving stem cells.

Generally, cells are seeded in a plastic flask or a glass dish or flask and cultured in an incubator. The inside of this incubator is set, for example, at a carbon dioxide concentration of 5%, a temperature of 37°C and a humidity of 60 % or higher, and maintained in an environment suitable for cell culture. In addition, medium is replaced once a day or at intervals of several days in order to provide nutrients to cells in the incubator while maintaining a pH suitable for cell culture.

To observe the cells being cultured by the above-described method, the plastic flask or the glass dish or flask is taken out of the incubator, and the cells therein are observed using an inverted microscope such as a phase contrast microscope.

However, this conventional method for observing cells in culture has a problem in that the activity of the cells is damaged, because the observation of the cells should be performed as quickly as possible and the cells should be returned to the incubator after the completion of observation. Moreover, there is a problem in that, because the activity of the cells is unstable, it is difficult to achieve accurate evaluation. Furthermore, if the cells are taken out of the incubator, contamination of the cells with toxic substances cannot be avoided. In addition, because the cells being cultured are generally colorless and transparent, these cells are difficult to observe with a transmission electron microscope.

Furthermore, in order to observe or image cells in culture according to the conventional method, both the incubator and the cell culture dish or flask should be opened, and for this reason, culture conditions are destroyed, making it impossible to observe or image cell culture in real time. In addition, the observation or imaging of cells relies on the skill of observers, variation in measurements between the observers occurs.

Thus, to observe in real-time the culture of, for example, stem cells whose differentiation process is necessarily required to be analyzed and monitored, the development of a new system, which can overcome the problems of conventional systems and easily observe or image a cell culture process in real time, has been required.

Accordingly, the present inventors have manufactured a novel system for analyzing cells and monitoring cell culture, and have found that the manufactured system can simultaneously analyze many types of cells under various conditions, can microscopically observe entire wells or can microscopically observe the region of interest of a single cell at high magnifications, can display dynamic cell images in real time, can overcome the problem of contamination during analysis, and can also remotely check the state of culture, thereby completing the present invention.

### Prior Art Documents

Korean Patent Laid-Open Publication No. 10-2012-0027359.

### DISCLOSURE

### Technical Problem

It is an object of the present invention to provide a novel system that overcomes the problems of conventional systems and enables real-time cell analysis and cell culture monitoring in an easy manner.

### Technical Solution

To achieve the above object, the present invention provides a system for analyzing cells or monitoring cell culture, comprising:
a light source unit configured to irradiate light downward;
a cell culture unit disposed below the light source unit and configured to mount thereon a cell culture vessel containing a sample and to perform culture of cells of the sample;
M x N matrix camera modules disposed below the cell culture unit and configured to observe an image of the cells, wherein M and N are each a natural number of 2 or more; and
a control unit configured to control the operation of a portion or all of the M x N matrix camera modules so as to observe a portion or all of the cells of the cell culture vessel mounted on the cell culture unit.

The present invention also provides a method for analyzing cells or monitoring cell culture using the system for analyzing cells or monitoring cell culture.

Other features and embodiments of the present invention will be more apparent from the following detailed description and the accompanying drawings.

### Brief Description of Drawings

FIG. 1 is a perspective view showing an example of a system for analyzing cells and monitoring cell culture according to the present invention.
FIG. 2 is an exploded view of the system shown in FIG. 1.
FIG. 3 is a schematic view showing an example of a camera module according to the present invention.
FIG. 4 shows an example of an ultra-compact imaging array having 96 camera modules for observation of a 96-well cell culture plate.
FIG. 5A shows the case in which only a partial region of a cell culture plate is observed (some camera modules corresponding to a green portion are switched on and the region corresponding thereto is observed), and FIG. 5B shows the case in which the entire region of a dish is observed.
FIG. 6 illustrates cell culture vessels, including 6/12/24/48/96 well plates, circular plates such as 35/60/100 Φ dishes, and culture flasks such as T25/75 flasks.
FIG. 7 shows a pH indication algorithm which is set in a system of the present invention so as to perform pH measurement based on a change in the color of a medium in a cell culture vessel.
FIG. 8 depicts photographs and a graph, which show the results obtained by measuring the morphological change and behavior of the cells of interest at indicated time points by use of a system for analyzing cells and monitoring cell culture according to the present invention.

### Mode for Invention

Unless otherwise defined, all technical and scientific terms used herein have the same meaning as commonly understood by those skilled in the art to which the present invention pertains. Generally, the terms used herein are those well known to and commonly used by those skilled in the art.

In one aspect, the present invention is directed to a system for analyzing cells or monitoring cell culture, comprising:
a light source unit configured to irradiate light downward;
a cell culture unit disposed below the light source unit and configured to mount thereon a cell culture vessel containing a sample and to perform culture of cells of the sample;
M x N matrix camera modules disposed below the cell culture unit and configured to observe an image of the cells, wherein M and N are each a natural number of 2 or more; and
a control unit configured to control the operation of a portion or all of the M x N matrix camera modules so as to observe a portion or all of the cells of the cell culture vessel mounted on the cell culture unit.

The system for analyzing cells or monitoring cell culture according to the present invention may be used for observation of not only cells in culture for use in regenerative medicine, for example, embryonic stem (ES) cells that are undifferentiated stem cells present in fertilized eggs, or undifferentiated stem cells present in each organ of an already formed body, for example, somatic stem cells such as hematopoietic stem cells or neural tube cells, but also all biological substances which are required to be analyzed and monitored in real time. Preferably, the system for analyzing cells or monitoring cell culture according to the present invention has a compact size such that it can be placed in a CO₂ incubator. Herein, the system for analyzing cells or monitoring cell culture according to the present invention may be provided integrally with a CO₂ or may be docked in a CO₂ incubator upon use. Alternatively, if the system of the present invention is not intended to use for live cell kinetic imaging assays, it can be placed outside an incubator so that it can be used like a conventional optical microscope, and in this case, it can perform high-throughput screening (HTS) in a better manner than a conventional optical microscope.

Hereinafter, preferred embodiments of the present invention will be described in further detail with reference to the accompanying drawings.

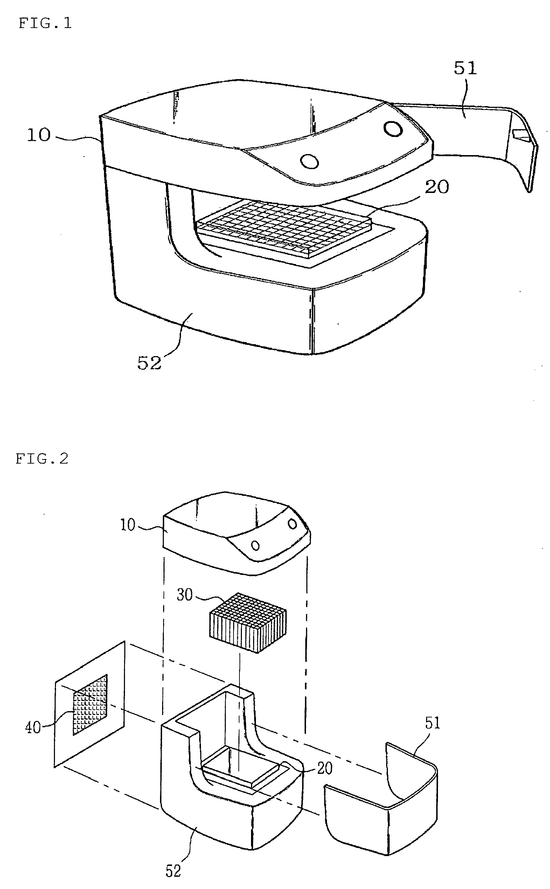

FIG. 1 is a perspective view showing an example of a system for analyzing cells and monitoring cell culture according to the present invention, and FIG. 2 is an exploded view of the system shown in FIG. 1. As shown therein, the system for analyzing cells and monitoring cell culture according to the present invention comprises: a light source unit 10 configured to irradiate light downward; a cell culture unit 20 disposed below the light source unit and configured to mount thereon a cell culture vessel containing a sample and to perform culture of cells in the sample; an M x N matrix (e.g., 96) camera modules 30 disposed below the cell culture unit and configured to image the cells; a control unit 40 configured to control the operation of a portion or all of the M x N matrix camera modules so as to observe a portion or all of the cells of the cell culture vessel mounted on the cell culture unit; and a housing unit 51 and 52 surrounding the light source unit, the cell culture unit, the camera modules and the control unit.

A light source that is used in the light source unit 10 may be any one selected from among organic field effect light-emitting elements, LEDs, FEDs, tungsten lamps, halogen lamps and mercury lamps. The organic field effect light-emitting element or LED may be composed of a sheet light source that emits UV, or may comprise an array of RGB pixels or an array of white pixels having three wavelengths so as to operate a pixel having a desired wavelength. Herein, the light source unit may advantageously comprise filters configured to select a certain type of light depending on the characteristics of the samples to be analyzed. In addition, the intensity of the light source can be controlled so as to a certain intensity depending on the characteristics of the sample to be analyzed.

Below the light source unit 10, a cell culture unit 20 configured to mount thereon a cell culture vessel containing a sample and to perform culture of cells in the sample is disposed. Below the cell culture unit 20, M x N matrix camera modules 30 are disposed, wherein M and N are each a natural number of 2 or more.

The camera modules 30 photograph an image of the cells (culture) of the sample and transmit the image to a computer by any appropriate communication means (wire or wireless communication). In the present invention, a microscope may be provided in the front of the camera modules to observe cells at a desired enlarged magnification. Herein, the camera modules can digitally receive the sample image provided by the microscope.

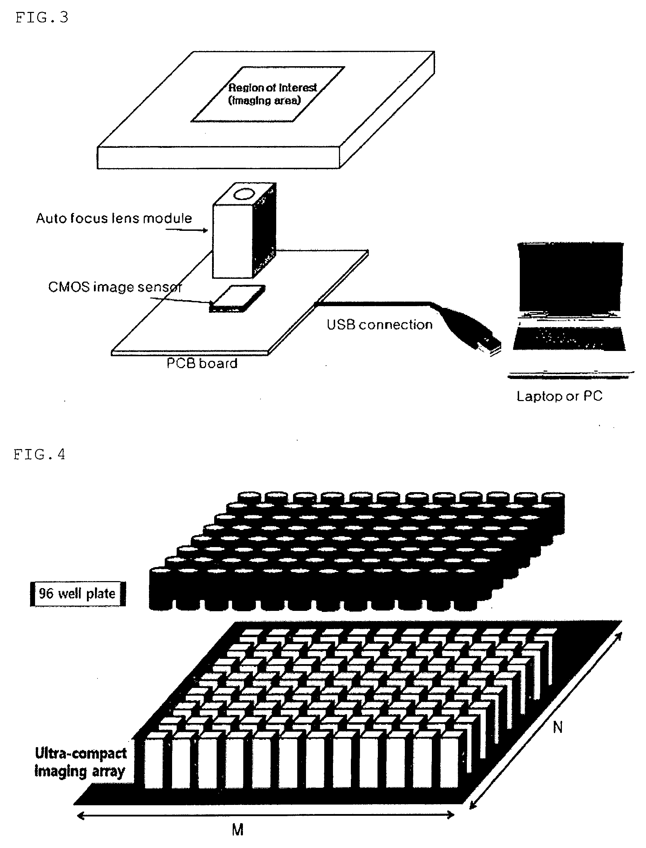

As shown in FIG. 3, the camera module according to the present invention may comprise a lens such as an autofocusing lens module or a passive focusing lens, and an image sensor such as a CMOS image sensor. The system according to the present invention comprises a plurality of the camera modules such that a certain space, that is, the region of interest, can be imaged by a single camera module.

In the present invention, the camera modules may be arranged in an M x N matrix, wherein M and N are each a natural number of 2 or more, preferably a natural number ranging from 2 to 64, more preferably a natural number ranging from 2 to 32. For example, 12 x 8 matrix (i.e., 96) camera modules may be arranged.

As used herein, the term "MxN matrix" means that M x N camera modules are arranged in a compact manner as shown in FIG. 4 to form a matrix. Specifically, it means that camera modules are arrayed at a high density.

Because the system according to the present invention comprises the camera modules 30 arranged in a matrix configuration, it can provide high-throughput imaging information. Preferably, as shown in FIG. 4, the system according to the present invention may comprise a highly compact array of 96 camera modules corresponding to 96 wells of a 96-well plate, a commercial cell culture plate which is widely used in the art. Accordingly, in an embodiment of the present invention, the system comprises 96 camera modules.

The camera modules that are used in the present invention may include various photo sensors, preferably a CMOS image sensor, a CCD image sensor or the like.

Meanwhile, the M x N matrix camera modules 30 may be disposed above or below the cell culture unit 20 without providing a separate light source unit 10 so that they can image the cells (culture) of the cell culture vessel on the cell culture unit 20.

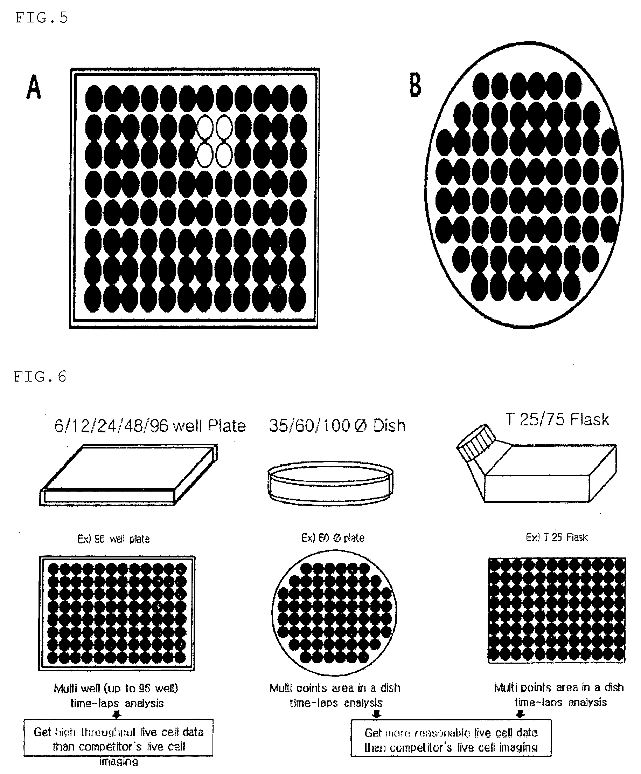

The system according to the present invention comprises a control unit 40 configured to control the operation of a portion or all of the camera modules arranged in an M x N (e.g., 96) matrix so as to image a portion or all of the cells of the cell culture vessel mounted on the cell culture unit, and thus can perform macroscopic observation (for example, analysis of the entire region of a single well of a 96-well cell culture plate) or microscopic observation (for example, analysis of a portion of a single well of a 96-well cell culture plate). In other words, it is possible to observe the entire region of a single well at a low magnification and observe the region of interest of a single well. FIG. 5 shows an embodiment of the present invention. As shown in FIG. 5A, only a partial region (green portion) of a cell culture plate can be selectively observed. Alternatively, as shown in FIG. 5B, the entire region of a cell culture dish can also be observed. In other words, according to the present invention, the camera modules are arranged in an M x N matrix, and thus only camera modules corresponding to a desired portion depending on the purpose of observation can be selectively switched on so that the desired portion can be observed.

A cell culture vessel containing a sample is mounted on the cell culture unit 20. The cell culture vessel is not specifically limited, as long as it contains the sample to be analyzed or monitored and enables cells to be directly cultured therein. Preferably, the cell culture vessel may be a plate having O x P wells, a dish or a flask. Herein, O and P are each a natural number of 2 or more, preferably a natural number ranging from 2 to 64, more preferably a natural number ranging from 2 to 32. For example, a 6/12/24/48/96/1024 well plate may be used, or a circular plate such as a 35/60/100 ϕ dish or a culture flask such as a T25/T75 flask may be used (FIG. 6). In an embodiment of the present invention, a 96-well plate is used as the cell culture vessel. In the present invention, a multi-well plate is used so that many types of cells can be analyzed at the same time under various conditions.

The cell culture vessel is preferably a vessel which at least has light-transmitting bottom and top surfaces. For example, the cell culture vessel is made of a transparent glass or resin material and is configured to transmit light irradiated from the light source unit 10 so that the content in the cell culture vessel can be observed by the lenses of the camera modules 30.

In the present invention, the cell culture unit 20 provides a space in which the cells in the sample are cultured. It comprises a smooth plate on which the cell culture vessel can be placed. It may further comprise a shaking means enabling shaking culture, and the operation of the shaking means can be controlled by the control unit. In addition, the cell culture unit 20 may further comprise a means configured to move the cell culture unit in a horizontal or vertical direction so as to enable a desired region to be easily observed or allow the movement pathway of cells to be analyzed when cell analysis or cell culture monitoring is performed by the camera modules. Alternatively, the system according to the present invention may further comprise a means configured to move the observation unit (camera module array) in a horizontal or vertical direction in a state in which the cell culture unit is fixed.

In addition, the system according to the present invention may comprise a sensor unit for monitoring one or more culture conditions selected from among temperature, humidity, carbon dioxide concentration, oxygen concentration, pH and flow rate in the cell culture unit 20 so as to maintain suitable culture conditions in the cell culture unit 20. This sensor unit may be separately provided or may be composed of an existing sensor unit, for example, a sensor provided in the camera modules.

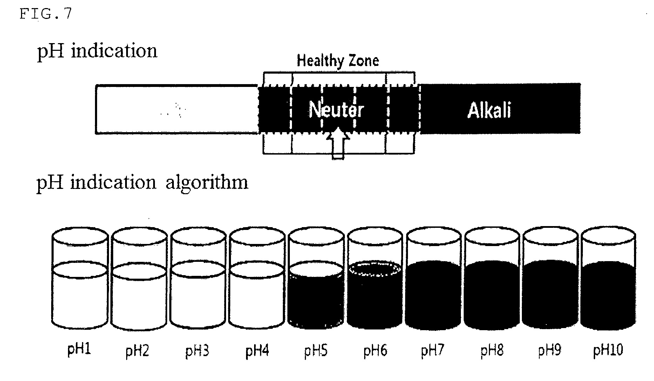

Particularly, the sensor unit may comprise a pH measurement unit configured to sense the pH in the cell culture unit. As shown in FIG. 7, a pH-dependent difference in color can be preset, and based on the preset difference, a change in the color of a culture can be monitored. Herein, pH conditions provide various information depending on cell culture environments. Preferably, an operator may set the system such that an alarm is given by a programmable control system (e.g., a server, a personal computer, a mobile device, etc.) or medium is replaced, when the pH is out of a zone selected as a healthy zone. In this case, contamination of the sample can be prevented by minimizing the exposure of the sample to an external environment. Preferably, the system according to the present invention may comprise a medium replacement unit configured to measure the timing of replacement of the medium depending on the pH information and to automatically discharge the medium from the cell culture vessel and also supply fresh medium to the cell culture vessel. As used herein, the expression "programmable control system" means a programmable control device including a computer. For example, the programmable control system may be a server, a personal computer, a mobile device or the like, but is not limited thereto, and may be any device or system that can be programmed to exchange information with the system of the present invention by two-way communication.

In the present invention, the control unit 40 is configured to exchange information with at least one programmable control system (e.g., a server, a personal computer, a mobile device or the like) by two-way communication, and may be wire- or wireless-connected with the programmable control system. In other words, the control unit 40 transmits digital image information, acquired by the camera modules 30, to a programmable control system (e.g., a server, a personal computer, a mobile device or the like) wire- or wireless-connected thereto, and this control unit 40 may be controlled by a computer through wire or remote control. In addition, the camera module, the light source unit and the like can be automatically controlled.

As used herein, the expression "exchange information by two-way communication" means that real-time information for cells in the cell analysis or culture system or values for culture conditions (for example, temperature, humidity, pH in medium or the concentration of nutrients in medium, etc.) are provided through the control unit and that the programmable control system connected to the control unit can instruct the control system to perform desired action, for example, medium replacement, temperature rise, adjustment of pH in medium, etc., by the cell analysis or culture system of the present invention.

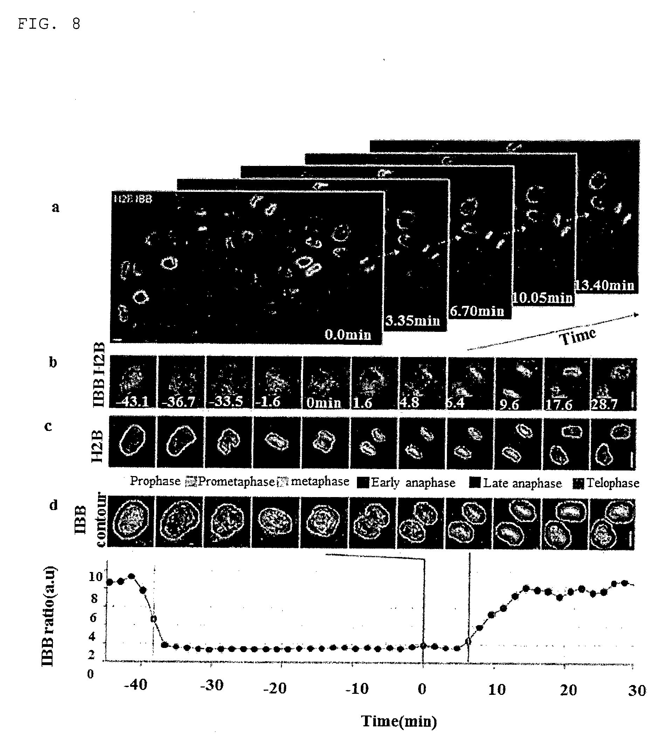

In an example of the present invention, as shown in FIG. 8, it was found that the use of the cell analysis or cell culture monitoring system according to the present invention enables the intermittent or time-lapse tracking of morphological changes and cell behaviors such as cell proliferation and cell growth in a desired time unit or for a desired period in a scheduled or captured manner, and as a result, the observation of a cell culture and the real-time analysis of cell division can be very easily achieved.

In other words, the cell analysis or cell culture monitoring method according to the present invention is easily used to analyze changes in cells after experiments in various fields, including the examination of reactivity of cancer cell treatment drugs, new drug development, etc. Particularly, it could be seen that the cell analysis or cell culture monitoring method according to the present invention is very advantageous for stem cell culture analysis in which it is necessary to analyze and track a cell differentiation process in order to find the mechanism of action of cells or a cell derivation method.

Accordingly, in another aspect, the present invention is directed to a method for analyzing cells or monitoring cell culture using the cell analysis or cell culture monitoring system. The method according to the present invention may comprise the steps of: culturing a sample in the cell culture unit of the cell analysis or cell culture monitoring system; and acquiring at least one digital image of the sample.

The cell analysis or cell culture monitoring system according to the present invention can operate in such a manner that it communicates with a programmable control system by two-way communication. For example, the method according to the present invention may comprise a step of transmitting the at least one acquired digital image to a programmable control system (e.g., a server, a personal computer, a mobile device, etc.) by wire or wireless communication. In addition, the method according to the present invention may comprise controlling the cell analysis or cell culture monitoring system by use of a programmable control system (e.g., a server, a personal computer, a mobile device, etc.) by wire or wireless communication.

The method according to the present invention may comprise using a multi-docking system so as to control two or more cell analysis or cell culture monitoring systems by a single computer. This multi-docking system can advantageously provide high-throughput analysis such as CLIA analysis based on a conventional 96-well plate.

In addition, the method according to the present invention may preferably comprise performing monitoring service according to a programmed schedule so as to enable automatic image capture and storage. In other words, it is possible to observe cells (culture) according to a programmed schedule for a long term.

### Industrial Applicability

As described above, the system for analyzing cells and monitoring cell culture according to the present invention comprises M x N matrix camera modules disposed below the cell culture unit in which a cell culture vessel is received, and thus can simultaneously analyze changes (caused by various changes in culture conditions or external environments) in the cells being cultured in a multi-well cell culture vessel such as a 96-well plate. Particularly, the system according to the present invention enables a desired portion to be observed by selectively switching on only camera modules corresponding to the desired portion. In addition, the system according to the present invention can microscopically observe entire wells or can microscopically observe the region of interest of a single cell at high magnifications, can display dynamic cell images in real time, can overcome the problem of contamination during cell analysis and culture, can remotely check the state of culture in real time, and can be controlled to perform a certain operation, if necessary.

Although the present disclosure has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereof.

### Description of Reference Numerals used in the Figures

- 10:: light source unit;
- 20:: cell culture unit;
- 30:: camera modules;
- 40:: control unit;
- 51:: door;
- 52:: housing.

## Claims

1. A system for analyzing cells or monitoring cell culture, comprising:
a light source unit configured to irradiate light downward;
a cell culture unit disposed below the light source unit and configured to mount thereon a cell culture vessel containing a sample and to perform culture of cells of the sample;
M x N matrix camera modules disposed below the cell culture unit and configured to observe an image of the cells, wherein M and N are each a natural number of 2 or more; and
a control unit configured to control operation of a portion or all of the M x N matrix camera modules so as to observe a portion or all of the cells of the cell culture vessel mounted on the cell culture unit.

2. The system of claim 1, wherein M and N are each a natural number ranging from 2 to 64.

3. The system of claim 1, wherein the cell culture vessel is a plate having O x P wells, a dish, or a flask, wherein O and P are each a natural number of 2 or more.

4. The system of claim 3, wherein the cell culture vessel is a 96-well plate.

5. The system of claim 1, wherein the cell culture unit comprises a shaking culture means.

6. The system of claim 1, further comprising a means configured to move the cell culture vessel in a horizontal or vertical direction or a means configured to move the camera modules in a horizontal or vertical direction in a state in which the cell culture vessel is fixed.

7. The system of claim 1, further comprising a sensor unit configured to monitor one or more culture conditions selected from among temperature, humidity, carbon dioxide concentration, oxygen concentration, pH and flow rate.

8. The system of claim 7, wherein the sensor unit comprises a pH measurement unit configured to measure pH in the cell culture vessel.

9. The system of claim 7, further comprising a medium replacement unit configured to discharge medium from the cell culture vessel according to a detected signal output from the sensor unit and to supply fresh medium to the cell culture vessel.

10. The system of claim 1, wherein the control unit is wire- or wireless-connected with one or more programmable control systems.

11. The system of claim 1, which has a size suitable for placement in a CO₂ incubator.

12. A method for cell analyzing cells or monitoring cell culture using a system for analyzing cells or monitoring cell culture according to any one of claims 1 to 11.

13. The method of claim 12, comprising the steps of:
culturing a sample in the system for analyzing cells or monitoring cell culture; and
acquiring at least one digital image of the sample.

14. The method of claim 13, further comprising a step of transmitting the at least one acquired digital image to a programmable control system by wire or wireless communication.

15. The method of claim 13, which is controlled by one or more programmable control systems.

16. The method of claim 13, further comprising a step of measuring pH of the cell culture unit of the system for analyzing cells or monitoring cell culture, and generating an alarm when the measured pH is out of a preset range.

17. The method of claim 12, wherein two or more systems for analyzing cells or monitoring cell culture are controlled by a single programmable control system.

18. The method of claim 12, wherein cell analysis or cell culture monitoring is performed according to a programmed schedule.
